# EUROPEAN PATENT SPECIFICATION

(11) **EP 3 413 828 B1**
(45) Date of publication and mention of the grant of the patent: **02.09.2020**
(21) Application number: 17712225.6
(22) Date of filing: 07.02.2017
(51) Int. Cl.: A61B 50/30, A61L 2/24, A61B 90/90, A61B 90/98, A61L 2/20

(54) **METHOD FOR THE PREPARATION AND PACKAGING OF A STERILE PROCEDURE PACK OR KIT FOR OPERATING ROOM USE**
VERFAHREN ZUR HERSTELLUNG UND VERPACKUNG EINER PACKUNG ODER EINES KIT FÜR STERILE VERFAHREN ZUR VERWENDUNG IN OPERATIONSSÄLEN
PROCÉDÉ POUR LA PRÉPARATION ET LE CONDITIONNEMENT D'UN EMBALLAGE OU D'UN KIT POUR INTERVENTION STÉRILE DESTINÉ À UNE UTILISATION EN SALLE D'OPÉRATION

(30) Priority: 10.02.2016 IT UB20160638
(43) Date of publication of application: 19.12.2018
(73) Proprietor: Servizi Ospedalieri S.p.A., 44122 Ferrara (IT)
(72) Inventor: FRANCESCHINI, Alberto, 44122 Ferrara (IT)
(74) Representative: Sandri, Sandro
(86) International application number: PCT/IB2017/050643
(87) International publication number: WO 2017/137884

(56) References cited:
- WO-A1-2014/014658
- WO-A2-2006/086603
- WO-A2-2007/146739
- US-A- 5 025 920
- US-A1- 2011 313 894
- US-A1- 2013 269 713

## Description

### FIELD OF APPLICATION

The present invention relates to a method for the preparation and packaging of a sterile procedure pack or kit for operating room use.

More particularly, the present invention relates to a method for the preparation and packaging of a procedure pack or kit that contains within it a series of medical devices made sterile and immediately usable in the operating room after said pack has been opened.

The invention further relates to a sterile pack or kit that contains a series of health devices immediately usable in the operating room after said pack has been opened.

The invention is mainly applicable in the field of the supply of advanced products and services in the health field.

### PRIOR ART

Inside hospital operating rooms kits of products and normally used that are supplied inside packs made sterile and delivered sterile to the hospital operators.

Packs of sterile products have always been split into two large categories, the technical requirements of which are considered substantially incompatible.

Within a first category all products made of reusable technical textiles (hereinafter indicated by the acronym RTT) must be considered, which are normally sterilised by steam in an autoclave, and whose traceability must necessarily be guaranteed.

On the other hand, within a second category products made of single-use technical textiles (hereinafter indicated by the acronym NWF) are considered, as well as a series of different accessories such as, for example, syringes, needles, extensions, bowls, scalpels, tweezers, valves, etc. for which the sterilisation operation is performed by treatment in an autoclave using gas, in particular ethylene oxide (hereinafter indicated by the acronym ETO).

The two methods implemented respectively for the sterilisation of health aids for the operating room belonging to the two mentioned categories differ greatly from one another.

In fact, steam sterilisation takes place using saturated steam as a sterilising agent and creating, within a sterilisation chamber, predetermined physical pressure and temperature conditions for a given time period, and the sterilisation cycle envisages subsequent steps of creating a vacuum, injecting steam, sterilisation and drying. During every sterilisation cycle, the sensors connected to the sterilisation chamber of the autoclave send the data related to the physical parameters of the process, i.e. temperature and pressure, to an electronic control system.

By way of example, typical technical parameters of the sterilisation processes applied for the sterilisation of RTTs are indicated in the following table:

| T° | Time | Absolute pressure bar | Absolute pressure mbar | Absolute pressure kbar | Relative pressure bar | Relative pressure mbar | Relative pressure kbar |
|---|---|---|---|---|---|---|---|
| 134°C | ≥ 3 min | 3.042 | 3042 | 304.2 | 2.042 | 2042 | 204.2 |
| 137°C | ≥ 3 min | 3.415 | 3415 | 341.5 | 2.415 | 2415 | 241.5 |

As mentioned above, the ETO sterilisation method takes place using ethylene oxide as a sterilising agent.

The inactivation of the micro-organisms by the ETO is influenced by various factors which are: concentration of ethylene oxide, temperature, relative humidity and exposure time.

The anti-microbial action depends on the dose. If the temperature and relative humidity values are kept constant, by increasing the concentration of the gas the inactivation rate of the micro-organisms increases and therefore the inactivation time is reduced.

In order to be able to exert its sterilising effect, the ETO needs humidity. The optimal relative humidity (rh) value required in the sterilisation site is 35%, but in common practice the rh values in autoclaves are a bit higher, 40-60%.

The fundamental stages of a sterilisation cycle with ethylene oxide ETO are:
1. Elimination of air through vacuum;
2. Humidification to reach the desired relative humidity value and preconditioning of the load using steam;
3. Inlet of the sterilising gas. The addition must not make the temperature change. The pressure value that corresponds to the desired concentration of ETO must be reached;
4. Exposure to the gas for the predefined time. The time may be very variable - from tens of minutes to a few hours - according to the conditions used;
5. Evacuation of the gas;
6. Forced degassing;
7. Final vacuum cycles alternating with the inlet of sterile air/inert gas.

The material must be arranged in the sterilisation chamber so that everything can be easily permeated by steam and gas. After the product has been loaded and heated to 50-60°C partial vacuum is created for eliminating air, the steam is injected into the sterilisation chamber so as to reach a rh of 40-60%, the sterilising gas is introduced into the sterilisation chamber, reaching operating pressures according to the concentration of the mixture but not below 48 kpa (lower than atmospheric pressure to prevent diffusion to the outside). After the required sterilisation time, the sterilising gas is evacuated by creating the vacuum.

Finally, the products must be subjected to a degassing step to eliminate any particles of ETO. The load is subjected to a standstill time in a controlled temperature environment inside which a biological indicator is placed; the outcome of its reading determines the release of the sterile product.

The present description facilitates the understanding of how, according to the products and materials to be sterilised, the market has proposed different sterilisation processes that have led to the provision of different kits of materials for operating room use, some sterilised with steam and others sterilised with ETO, and the conviction has become widespread that it is impossible to assemble a combined kit that allows sterile materials made of reusable technical textiles RTT and materials made of single-use technical textiles NWF and various disposable accessories to be inserted in a single sterile pack. This sort of technical prejudice is a source of high preparation and supply costs for the packs or kits of sterilising materials for operating room use.

Document WO 2014/014658 A1 discloses a custom procedure kit comprising a plurality of medical-procedure tools and a cover that is wrapped around the plurality of medical-procedure tools to form wrapped tools. An outer container is fully disposed about the wrapped tools and at least one sheet disposed between the outer container and the wrapped tools. Some or all of the medical-procedure tools in the kit may be sterile or non-sterile.

Document WO 2006/086603 A2 discloses a multiplex system for identifying the presence of surgical sponges and other implements in a surgical wound employing a plurality of discrete sensing systems. Detection modalities can include marker tags, RFID markers, systems for detecting metal objects.

Document WO 2007/146739 A2 discloses methods for sterilizing packaged or unpackaged medical devices such as drug- and/or polymer-coated stents. The method involves placing a packaged or unpackaged medical device into a pressure-, temperature- and humidity-controlled environment and exposing the device to a liquid or gaseous sterilizer such as ethylene oxyde for a given period of time.

Document US 2011/313894 A1 discloses a wrapped surgical pack including a plurality of adhesive bindings that retain an identification device against an outer wrap of the wrapped surgical pack. The identification device is capable of being read by a remote reader.

### DESCRIPTION OF THE INVENTION

The invention is defined in claim 1. Preferred embodiments are defined in the dependent claims. The present invention sets out to provide a solution to the drawbacks and disadvantages typical of the prior art and therefore to provide a method for the packaging of a combined sterile pack or kit for operating room use within which products or materials made of RTT and products or materials made of NWF are positioned, as well as a series of accessories whose sterility must be guaranteed in the operating room.

This is obtained through a method for the packaging of a sterile procedure pack or kit for operating room use having the characteristics described in claim 1.

The dependent claims outline particularly advantageous embodiments of the method according to the invention.

Experiments performed by the applicant, in contrast with common practice, have been able to demonstrate how products or materials made of reusable technical textiles RTT, which have historically only be sterilised by steam in an autoclave, under certain conditions can also be sterilised through the method that uses ethylene oxide ETO. It follows that, under certain conditions and when the traceability of the reusable technical textile products is guaranteed, it is possible to prepare and package a combined pack containing both RTT items and single-use textiles or other items of common operating room use. The result is a sterile procedure pack of medical devices that allows the operating room personnel to be supplied with the material required for:
- equipping the operating field according to the indications of the particular surgical procedure involved so as to prevent risks of surgical infection through appropriately conformed equipped or simple drapes and/or protecting the patient and operators from risks of cross contamination through white coats;
- containing (where envisaged) disinfecting solutions for the patient's skin prior to the surgical incision (e.g. bowls);
- performing dressings;
- performing certain steps of the surgical procedure (e.g. scalpels and syringes).

In this way, substantial economic, time and environmental sustainability advantages are obtained.

The method for the preparation and packaging of a sterile procedure pack or kit for operating room use comprises the following successive steps:
a) washing and technical folding of reconditioned reusable technical textile RTT garments and microchip reading and saving of traceability data through appropriate electronic means; and/or
a') washing and folding of new reusable technical textile RTT garments and microchip association and saving of traceability data through appropriate electronic means;
b) provision of a controlled contamination environment for the preparation of a procedure pack or kit for operating room use;
c) provision, within the environment referred to in paragraph b), of said reusable technical textile RTT garments equipped with microchips, of further single-use textile NWF garments, as well as accessories for the operating room, such as, by way of non-limiting example, extensions, bowls, caps, scalpels, syringes, needles, tweezers, valves, cotton gauze, etc.
d) preparation, packaging and closing of a procedure pack containing the materials or products referred to in paragraph c) within said controlled contamination environment;
e) sterilisation of the procedure pack referred to in paragraph d) through treatment with ethylene oxide ETO gas for a predetermined time, at a predetermined temperature, predetermined pressure and predetermined humidity level;
f) natural and/or forced evacuation of the ETO sterilising gas from said pack for a predetermined time and control of the potential presence of ETO inside said procedure pack;
g) reading via multireader tunnel or antenna of the data contained in the microchip of the RTT material contained inside the pack;
h) storage of said pack inside a sterile storage area;
i) release of the sterile procedure pack and delivery to users in the operating room;
j) collection of the RTT material after use for appropriate reconditioning.

In compliance with a preferred embodiment of the method according to the present invention for the preparation of the pack, the materials are arranged and inserted into an envelope made of synthetic material. Furthermore, according to a further particular embodiment, such synthetic material envelope is inserted inside a packaging box, advantageously made of corrugated cardboard, which is reclosed and equipped with a series of labels indicating the contents of the pack and the batch of packs being produced.

According to a further preferred embodiment, the reusable technical textile RTT material subject to washing is dried and inserted into tanks or closed cabinets for being taken to said controlled contamination environment.

The result of such method for the preparation, packaging and sterilisation of a procedure pack for operating room use comprises an object never created prior to the method according to the present invention, and that constitutes total novelty in the world of providing services for operating rooms, i.e. a procedure pack sterilised through a sterilisation method based on ethylene oxide ETO which contains inside it both material made of reusable technical textile RTT, reconditioned, and single-use technical textile NWF, as well as accessories for the operating room made of different metal and/or synthetic materials.

### ILLUSTRATION OF THE DRAWINGS

Further characteristics and advantages of the invention will be evident from reading the following description of an embodiment of the invention by way of non-limiting example with the aid of the figures illustrated in the appended tables of drawings, in which:
- figures 1 and 2 illustrate, in a schematic perspective view, the various components for realising a procedure pack to be sterilised according to the invention for performing a caesarian section; and
- figures 3 and 4 illustrate, in a schematic perspective view, the various components for realising a procedure pack to be sterilised according to the invention for performing an angiography.

### DESCRIPTION OF AN EMBODIMENT OF THE INVENTION

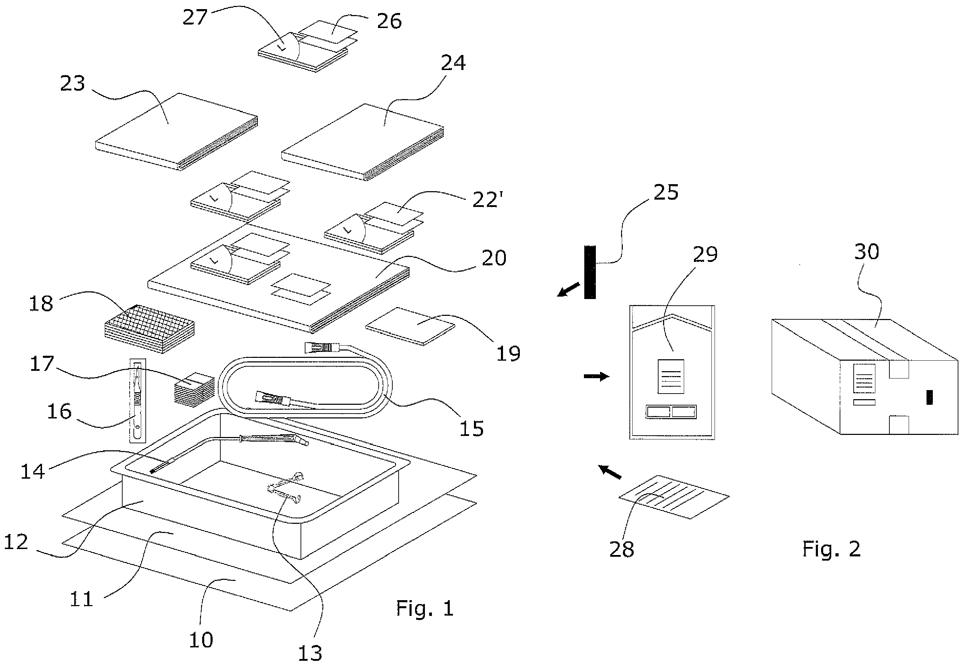

In figures 1 and 2, an operating room kit for performing a caesarian section comprises, within a controlled contamination environment (otherwise known as a "cleanroom"), a sheet of crepe paper 10, an operating room table drape 11 on which a tray 12 is positioned.

The following components are positioned within said tray 12:
- an umbilical chord clamp 13;
- a Yankauer cannula 14;
- a suction tube 15;
- a scalpel 16;
- small sheets of gauze 17;
- laparotomy gauze 18;
- an adhesive strip 19.

On top of these components an obstetric drape 20 is placed, on which, in turn, wipes 21 and white coats 22, 22' are arranged tidily. A drape 23 made of RTT and a Mayo bag 24 are positioned thereon.

According to the invention, the above-listed components are then completely wrapped up by the said operating table drape 11 and the package is secured by chemical process indicating tape 25. Then, a further towel 26 and white coat 27 are positioned on the package. Everything is further wrapped with the said crepe paper sheet 10, secured with chemical process indicating tape 25 and equipped with a contents list 28. The kit thus prepared is then inserted into a special envelope 29 onto which an identification label is applied. The packaging is then completed by inserting two kits in envelopes as described above into a cardboard box 30 on which a label and a colour change chemical indicator are applied.

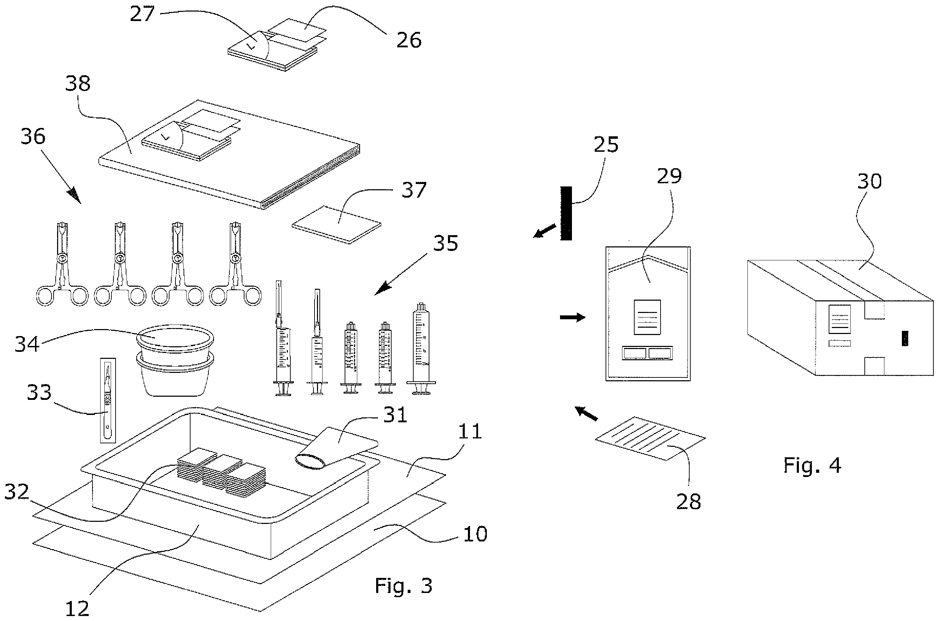

Figures 3 and 4 show the preparation and packaging of a further kit for the operating room for performing an angiography. It comprises, within a controlled contamination environment (otherwise known as a "cleanroom"), a sheet of crepe paper 10, an operating room table drape 11 on which a tray 12 is positioned.

The following components are positioned within said tray 12:
- a cap 31;
- gauze 32;
- a scalpel 33;
- bowls 34;
- various syringes 35 with and without needles;
- drape clamps 36;
- a RTT drape 37.

On top of these components an angiography drape 38, a white coat 39 and wipes 40 are positioned.

Everything is then wrapped up with the operating room table drape 11 and secured with the chemical process indicating tape 25.

Then, a further towel 26 and white coat 27 are positioned on the package. Everything is further wrapped with the said crepe paper sheet 10, secured with chemical process indicating tape 25 and equipped with a contents list 28. The kit thus prepared is then inserted into a special envelope 29 onto which an identification label is applied. The packaging is then completed by inserting two kits in envelopes as described above into a cardboard box 30 on which a label and a colour change chemical indicator are applied.

In relation to the reusable technical textile, in general a polyester mesh, polyester microfibre or trilaminate fabric is generally used, the latter being obtained through the lamination of two layers of polyester on the two sides of a membrane, generally a polytetrafluoroethylene PTFE membrane or a microperforated polyurethane PU membrane.

These products are inserted into the kit in the new condition (if used for the first time) simply washed to remove any manufacturing treatment residues, or in a condition previously subjected to chemical industrial/heat washing without ironing.

Once washed, such products are immediately inserted into tanks or closed cabinets in order to prevent recontamination.

Again, before being inserted into the kit, the reusable technical textile products are equipped with electronic identification codes and recorded (if being used for the first time) or simply recorded (if being reused).

The single-use textiles are normally comprised of non-woven fabric NWF in various versions according to the degrees of absorption that are intended to be used.

The accessories are generally comprised of objects made of synthetic plastic material, for example polypropylene or polyethylene, silicone, or stainless steel or carbon.

The envelopes are generally made of coupled polyethylene/TYVEK®, and the boxes of corrugated cardboard for packaging.

The procedure packs thus packaged are subsequently subjected to a sterilisation treatment using ethylene oxide ETO.

The fundamental stages of a sterilisation cycle with ethylene oxide are:
1. Elimination of air through vacuum;
2. Humidification to reach the desired relative humidity value and preconditioning of the load using steam;
3. Inlet of the sterilising gas. The addition must not make the temperature change. The pressure value that corresponds to the desired concentration of ETO must be reached;
4. Exposure to the gas for the predefined time. The time may be very variable - from tens of minutes to a few hours - according to the conditions used;
5. Evacuation of the gas;
6. Washing (forced degassing)
7. Final vacuum cycles alternating with the inlet of sterile air/inert gas.

The material must be arranged in the sterilisation chamber so that everything can be easily permeated by steam and gas. After the product has been loaded and heated to 50-60°C partial vacuum is created for eliminating air, the steam is injected into the sterilisation chamber so as to reach a rh of 40-60%, the sterilising gas is introduced into the sterilisation chamber, reaching operating pressures according to the concentration of the mixture but not below 48 kpa (lower than atmospheric pressure to prevent diffusion to the outside).

After the required sterilisation time, the sterilising gas is evacuated by creating the vacuum. Finally, the products must be subjected to a degassing step to eliminate any particles of ETO. The load is subjected to a standstill time in a controlled temperature environment inside which a biological indicator is placed; the outcome of its reading determines the release of the sterile product.

Experiments performed by the applicant have surprisingly been able to demonstrate how such sterilisation with ETO allows a suitable degree of sterilisation to be obtained also for reusable technical textiles, which were previously only sterilised with steam at high temperature. Therefore, the procedure packs according to the present invention allow both procedure packs for reusable technical textiles RTT and procedure packs for single-use technical textiles and operating room accessories to be replaced easily, by combining their best technical characteristics. In this way it is possible to achieve remarkable advantages both from an organisational and work safety point of view, as well as the value for money of the product to be supplied to operating rooms.

## Claims

1. A method for the preparation and packaging of a sterile procedure pack or kit for operating room use comprising the following work steps:
a) washing and folding of reconditioned reusable technical textile (RTT) garments and microchip reading and saving of traceability data through appropriate electronic means; and/or
a') washing and folding of new reusable technical textile (RTT) garments and microchip association and saving of traceability data through appropriate electronic means;
b) provision of a controlled contamination environment for the preparation of a procedure pack or kit for operating room use;
c) provision, within the environment referred to in paragraph b), of said reusable technical textile (RTT) garments equipped with microchips, of further single-use technical textile (NWF) garments, as well as typical accessories for the operating room, in particular extensions, bowls, caps, scalpels, syringes, needles, tweezers, valves, cotton gauze, etc.
d) preparation, packaging and closing of a procedure pack containing the materials or products referred to in paragraph c) within said controlled contamination environment;
e) sterilisation of the procedure pack referred to in paragraph d) through treatment with ethylene oxide gas (EtO) for a predetermined time, at a predetermined temperature, predetermined pressure and predetermined humidity level inside a sterilisation autoclave according to the following process:
i) inserting the closed procedure pack into said autoclave, heating it to a temperature comprised between 50°C and 60°C, and eliminating air by placing under vacuum;
ii) injecting steam and consequent humidification up to a desired relative humidity (rh) value comprised between 30% and 60%;
iii) inletting of ethylene oxide (EtO) sterilising gas without substantial temperature variations at a pressure of at least 48kPa;
iv) exposing said procedure pack to the gas for a predetermined time comprised between tens of minutes and a few hours;
v) evacuating the gas from the autoclave;
vi) forced degassing;
vii) carrying out final vacuum cycles alternating with the inlet of sterile air/inert gas;
f) reading via appropriate electronic means of the data contained in the microchip of the RTT material contained inside the pack.

2. The method according to claim 1, wherein the said electronic data reading means comprises multireader tunnels or antennae.

3. The method according to one of the preceding claims, wherein the reusable technical textile (RTT) material subject to prior washing is dried and inserted into tanks or closed cabinets for being taken to said controlled contamination environment.

4. The method according to one of the preceding claims, wherein the materials are arranged and inserted into an envelope made of synthetic material.

5. The method according to claim 4, wherein such synthetic material envelope is inserted inside a packaging box, advantageously made of corrugated cardboard, which is reclosed and equipped with a series of labels indicating the contents of the pack and the batch of packs being produced.

6. The method according to one of the preceding claims, wherein a biological indicator is inserted within each sterilisation cycle, the reading of which indicates the degree of sterilisation obtained through said method.

## Patentansprüche

1. Verfahren zur Herstellung und Verpackung eines Sterilverfahren-Pakets oder -Kits für einen Operationssaal, umfassend die folgenden Arbeitsschritte:
a) Waschen und Falten rekonditionierter wiederverwendbarer Kleidungsstücke aus technischen Textilien (RTT) und Lesen von Mikrochips und Speichern von Rückverfolgbarkeitsdaten mit geeigneten elektronischen Mitteln; und/oder
a') Waschen und Falten neuer wiederverwendbarer Kleidungsstücke aus technischen Textilien (RTT) und Assoziieren von Mikrochips und Speichern von Rückverfolgbarkeitsdaten durch geeignete elektronische Mittel;
b) Bereitstellen einer kontrollierten Kontaminationsumgebung für die Herstellung eines Verfahren-Pakets oder -Kits für den Einsatz im Operationssaal;
c) Bereitstellen, in der in Absatz b) genannten Umgebung, der genannten wiederverwendbaren Kleidungsstücke aus technischen Textilien (RTT), die mit Mikrochips ausgestattet sind, von weiteren Einweg-Kleidungsstücken aus technischen Textilien (NWF) sowie von typischem Zubehör für den Operationssaal, insbesondere Verlängerungen, Schalen, Kappen, Skalpellen, Spritzen, Nadeln, Pinzetten, Ventilen, Baumwollgaze usw.;
d) Herstellen, Verpacken und Verschließen eines Verfahren-Pakets, das die in Absatz c) genannten Materialien oder Produkte enthält, in der genannten kontrollierten Kontaminationsumgebung;
e) Sterilisieren des in Absatz d) genannten Verfahren-Pakets durch Behandlung mit Ethylenoxid- (Eto-) Gas für eine vorbestimmte Zeit, bei einer vorbestimmten Temperatur, einem vorbestimmten Druck und einem vorbestimmten Feuchtigkeitsniveau in einem Sterilisierungsautoklav gemäß dem folgenden Verfahren:
i) Einsetzen des verschlossenen Verfahren-Pakets in den genannten Autoklav, Erhitzen desselben auf eine Temperatur zwischen 50 °C und 60 °C, und Eliminieren von Luft durch Setzen unter Vakuum;
ii) Einspritzen von Dampf und entsprechendes Befeuchten bis auf einen gewünschten Wert der relativen Feuchtigkeit (RH) zwischen 30 % und 60 %;
iii) Einlassen von Ethylenoxid- (Eto-) Sterilisierungsgas ohne wesentliche Temperaturvariationen bei einem Druck von mindestens 48 kPa;
iv) Aussetzen des genannten Verfahren-Pakets an das Gas für eine vorherbestimmte Zeit zwischen einigen zehn Minuten und einigen Stunden;
v) Abführen des Gases aus dem Autoklav;
vi) zwangsweises Entgasen;
vii) Durchführen von Endvakuumzyklen, die mit dem Einlassen von steriler Luft/inertem Gas abwechseln;
f) Lesen der Daten, die in dem Mikrochip des in dem Paket enthaltenen RTT Materials enthalten sind, über geeignete elektronische Mittel.

2. Verfahren nach Anspruch 1, wobei das genannte elektronische Datenlesemittel Multireader-Tunnel oder Antennen umfasst.

3. Verfahren nach einem der vorhergehenden Ansprüche, wobei das wiederverwendbare Material aus technischen Textilien (RTT), das einem vorhergehenden Waschen unterzogen wird, getrocknet und in Tanks oder geschlossene Behälter eingebracht wird, um in die genannte kontrollierte Kontaminationsumgebung gebracht zu werden.

4. Verfahren nach einem der vorhergehenden Ansprüche, wobei die Materialien angeordnet und in eine Hülle aus synthetischem Material eingesetzt werden.

5. Verfahren nach Anspruch 4, wobei eine solche Hülle aus synthetischem Material in eine Verpackungsschachtel eingesetzt wird, die vorteilhafterweise aus Wellpappe besteht, die wieder verschlossen und mit einer Reihe von Etiketten versehen wird, welche den Inhalt des Pakets und die Charge der hergestellten Pakete angeben.

6. Verfahren nach einem der vorhergehenden Ansprüche, wobei ein biologischer Indikator in jedem Sterilisierungszyklus eingefügt wird, dessen Ablesung den Sterilisierungsgrad anzeigt, der durch das Verfahren erhalten wird.

## Revendications

1. Méthode de préparation et de conditionnement d'un nécessaire ou d'un kit de procédure stérile pour utilisation en salle d'opération comprenant les étapes de travail suivantes :
a) lavage et pliage des vêtements en textile technique réutilisable (RTT) reconditionnés et lecture de micropuce et enregistrement des données de traçabilité par des moyens électroniques appropriés ; et/ou
a') lavage et pliage de nouveaux vêtements en textile technique réutilisable (TTT) et association de micropuce et enregistrement des données de traçabilité par des moyens électroniques appropriés ;
b) fourniture d'un environnement à contamination contrôlée pour la préparation d'un nécessaire ou d'un kit de procédure destiné à être utilisé en salle d'opération ;
c) la fourniture, dans l'environnement visé au point b), desdits vêtements en textile technique réutilisable (RTT) équipés de micropuces, d'autres vêtements en textile technique à usage unique (NWF), ainsi que des accessoires typiques pour le bloc opératoire, notamment des rallonges, des cuvettes, des capuchons, des scalpels, des seringues, des aiguilles, des pinces, des valves, de la gaze de coton, etc.
d) la préparation, l'emballage et la fermeture d'un nécessaire de procédure contenant les matériaux ou produits visés au point c) dans ledit environnement à contamination contrôlée ;
e) stérilisation du nécessaire de procédure visé au point d) par traitement à l'oxyde d'éthylène gazeux (Et0) pendant un temps prédéterminé, à une température prédéterminée, une pression prédéterminée et un taux d'humidité prédéterminée dans un autoclave de stérilisation selon le procédé suivant :
i) insertion du nécessaire de procédure fermé dans ledit autoclave, chauffage de celui-ci à une température comprise entre 50°C et 60°C, et élimination de l'air par mise sous vide ;
ii) injection de vapeur et humidification consécutive jusqu'à une valeur d'humidité relative (rh) souhaitée comprise entre 30 % et 60 % ;
iii) l'introduction d'oxyde d'éthylène (Et0) gazeux stérilisant sans variations substantielles de température à une pression d'au moins 48kPa ;
iv) l'exposition dudit nécessaire de procédure au gaz pendant une durée prédéterminée comprise entre des dizaines de minutes et quelques heures;
v) l'évacuation du gaz de l'autoclave ;
vi) le dégazage forcé ;
vii) l'exécution de cycles de vide final en alternance avec l'entrée d'air stérile/gaz inerte ;
f) lire par des moyens électroniques appropriés des données contenues dans la micropuce du matériel RTT contenu dans l'emballage.

2. Méthode selon la revendication 1, dans laquelle lesdits moyens de lecture de données électroniques comprennent des tunnels ou des antennes à lecteurs multiples.

3. Méthode selon l'une des revendications précédentes, dans laquelle le matériau textile technique réutilisable (RTT) soumis à un lavage préalable est séché et inséré dans des cuves ou des armoires fermées pour être amené dans ledit environnement à contamination contrôlée.

4. Méthode selon l'une des revendications précédentes, dans laquelle les matériaux sont disposés et insérés dans une enveloppe en matière synthétique.

5. Méthode selon la revendication 4, dans laquelle une telle enveloppe en matière synthétique est insérée à l'intérieur d'une boîte d'emballage, avantageusement en carton ondulé, qui est refermée et équipée d'une série d'étiquettes indiquant le contenu du nécessaire et le lot de nécessaires en cours de production.

6. Méthode selon l'une des revendications précédentes, dans laquelle un indicateur biologique est inséré à l'intérieur de chaque cycle de stérilisation, dont la lecture indique le degré de stérilisation obtenu par ladite méthode.
